# EUROPEAN PATENT APPLICATION

(11) **EP 4 817 623 A1**
(43) Date of publication of application: **07.10.2026**
(21) Application number: 24897298.6
(22) Date of filing: 13.11.2024
(51) Int. Cl.: A61K 36/064, A23L 33/135, A61P 29/00, A61P 37/02, A61P 37/04, A61P 37/06, A61P 43/00, C12N 1/16

(54) **IMMUNOMODULATION AGENT**

(30) Priority: 30.11.2023 JP 2023202438
(71) Applicant: Nisshin Seifun Group Inc., Chiyoda-ku Tokyo 101-8441 (JP)
(72) Inventor: TAKAHASHI, Erika, Fujimino-shi, Saitama 356-8511 (JP); NODE, Junichi, Fujimino-shi, Saitama 356-8511 (JP); KIKUCHI, Yosuke, Fujimino-shi, Saitama 356-8511 (JP); MATSUO, Shuhei, Tokyo 174-8505 (JP); OMACHI, Atsuko, Tokyo 174-8505 (JP)
(74) Representative: Forstmeyer, Dietmar
(86) International application number: PCT/JP2024/040277
(87) International publication number: WO 2025/115611

(57) **Abstract**

Provided is an immunomodulatory agent containing yeast deposited with the National Institute of Technology and Evaluation under International Deposit No. NITE BP-04012. Preferably, the immunomodulatory agent is for regulating cytokine production. More preferably, the immunomodulatory agent regulates cytokine production in macrophage cells or dendritic cells. More preferably, the immunomodulatory agent modulates immunity via TLR2. Preferably, the cytokine is at least one selected from IL-1β, IL-6, IL-8, IL-10, IL-12, and TNF-α.

## Description

### Technical Field

The present invention relates to an immunomodulatory agent including yeast.

### Background Art

Macrophage cells and dendritic cells are at the forefront of innate immunity. Macrophage cells not only perform phagocytosis but also play a role in transmitting antigen information to T cells, which function as coordinators. Dendritic cells are antigen-presenting cells (cells that present information about substances identified as foreign substances to other cells) and also perform phagocytosis. It is widely known that stimulation of macrophage cells and dendritic cells by pathogens and other stimuli leads to the production of various cytokines, and that this is an important process in the immune response.

For example, it is known that bacterial cell components (such as lipopolysaccharide (LPS)) activate TLRs (toll-like receptors) present on the surface of macrophages and dendritic cells, resulting in the production of proinflammatory cytokines, such as TNF-α, IL-1β, and IL-6, which initiate inflammation. IL-8 is also a proinflammatory cytokine that functions to induce neutrophils and other granulocytes to migrate toward the site of infection and also to induce phagocytosis.

IL-10 and IL-12 are also produced by macrophage cells and similar cells that have been stimulated by LPS and other stimuli, and these cytokines are known to have immunostimulatory properties and also have anti-inflammatory properties. For example, IL-10 exhibits immunostimulatory properties, including stimulating the proliferation of thymocytes treated with IL-2 and IL-4, increasing the survival rate of B cells, and stimulating MHC class II expression. On the other hand, IL-10 can suppress immune responses by inhibiting the expression of IL-1α, IL-1β, IL-6, IL-8, TNF-α, GM-CSF, and G-CSF in activated monocytes and activated macrophages, and also suppresses the production of interferon-gamma (IFN-γ) by NK cells.

IL-12 is a heterodimeric cytokine composed of the p35 and p40 subunits, which are encoded by two distinct genes, IL-12A and IL-12B, respectively. The main functions of IL-12 are to induce the production of interferon-gamma by NK cells and T cells and to enhance the activity of NK cells and cytotoxic T cells. Furthermore, it is known that IL-12 acts on naive T cells (Th0) to induce differentiation into Th1 cells, thereby promoting interferon-gamma production mediated by Th1 cells and preferentially activating cell-mediated immunity. In addition, IL-12 has been reported to activate cell-mediated immunity by preferentially activating Th1 cells and suppressing Th2 cells through mechanisms such as IgE suppression. In light of this, attention has been focused on the application of IL-12 to the treatment of Th2-dominated immune disorders such as allergies, as well as to cancer and infectious disease therapy by activating cell-mediated immune functions.

As mentioned above, TLRs play a significant role in recognizing various pathogens and activating the immune system. Furthermore, among the TLRs, TLR2 is expressed on the surface of certain cells, such as macrophages, and plays a fundamental role in pathogen recognition and in the activation of innate immunity.

Yeast has been known to be used as an immunostimulant (see Patent Literature 1 and Patent Literature 2).

### Citation List

### Patent Literature

Patent Literature 1: JP 2004-107281A
Patent Literature 2: JP 2006-75039A

### Summary of Invention

Immunomodulatory function refers to a function that modulates the immune status to a favorable state. An excellent immunomodulatory function is capable of exerting immunoenhancing and/or immunosuppressive effects to maintain the immune status in a favorable state. There is a need for immunomodulatory agents exerting such excellent immunomodulatory effects.

As a result of in-depth research, the inventors of the present invention have found that a specific strain has excellent immunomodulatory effects, and have thus achieved the present invention.

The present invention is based on the above findings and provides an immunomodulatory agent comprising the yeast deposited with the National Institute of Technology and Evaluation under International Deposit No. NITE BP-04012.

According to the present invention, it is possible to provide an immunomodulatory agent exerting excellent immunomodulatory effects. The immunomodulatory agent of the present invention can exert excellent immunoenhancing and/or immunosuppressive effects.

### Brief Description of Drawings

[Fig. 1] Fig. 1 shows graphs illustrating the results of evaluating gene expression of various cytokines in macrophage-like cells using a powder of a specific yeast.
[Fig. 2] Fig. 2 shows graphs illustrating the results of evaluating gene expression of various cytokines in macrophage-like cells using an extract of the specific yeast.
[Fig. 3] Fig. 3 shows a graph illustrating the results of evaluating IL-6 gene expression in dendritic cell-like cells using the extract of the specific yeast.
[Fig. 4] Fig. 4 shows graphs illustrating the results of evaluating production levels of various cytokines in macrophage-like cells using the powder of the specific yeast.
[Fig. 5] Fig. 5 shows graphs illustrating the results of evaluating production levels of various cytokines in macrophage-like cells using the extract of the specific yeast.
[Fig. 6] Fig. 6 shows graphs illustrating the results of evaluating gene expression of various cytokines in macrophage-like cells in the presence of LPS using the powder of the specific yeast.
[Fig. 7] Fig. 7 shows graphs illustrating the results of evaluating gene expression of various cytokines in macrophage-like cells in the presence of LPS using the extract of the specific yeast.
[Fig. 8] Fig. 8 shows graphs illustrating the results of evaluating production levels of various cytokines in macrophage-like cells in the presence of LPS using the powder of the specific yeast.
[Fig. 9] Fig. 9 shows graphs illustrating the results of evaluating production levels of various cytokines in macrophage-like cells in the presence of LPS using the extract of the specific yeast.
[Fig. 10] Fig. 10 shows graphs illustrating the results of evaluating TLR protein expression levels in genetically modified HEK293 cells using the powder of the specific yeast.
[Fig. 11] Fig. 11 shows graphs illustrating the results of evaluating TLR protein expression levels in genetically modified HEK293 cells using the extract of the specific yeast.

### Description of Embodiments

Hereinafter, the present invention will be described based on preferred embodiments thereof.

The present invention provides an immunomodulatory agent containing yeast deposited with the National Institute of Technology and Evaluation (NITE) under International Deposit No. NITE BP-04012. This specific yeast was deposited with the National Institute of Technology and Evaluation on November 14, 2023, under the deposit number NITE P-04012, and was transferred to international deposit on September 11, 2024.

The yeast deposited with the National Institute of Technology and Evaluation under International Deposit No. NITE BP-04012 (hereinafter also referred to as "specific yeast") has the scientific name *Saccharomyces cerevisiae*. Its scientific properties and others are as follows.
1. Scientific properties: It forms colonies ranging from white to pale yellow on nutrient media containing carbon and nitrogen sources. Under an optical microscope, proliferation by budding is observed as the form of cell division. Distinction between mother and daughter cells is possible.
2. Taxonomic position: yeast - edible yeast (genus *Saccharomyces*)
3. Culture conditions
   (1) Medium name: YPD medium
   (2) Medium composition: 10 g of yeast extract, 20 g of peptone, and 20 g of glucose, per 1000 ml of medium
   (3) Medium pH: 4 to 7 (optimal pH: 5 to 6)
   (4) Medium sterilization conditions: 121°C for 10 minutes
   (5) Culture temperature: 30°C
   (6) Culture period: 2 to 3 days
   (7) Oxygen requirement: facultative anaerobic
4. Storage conditions:
   It can be stored by a freezing method.
   (1) Freezing conditions: -80°C
   (2) Protectant: 10 to 20% aqueous glycerol solution (15% is optimal)
   (3) Recovery rate after freezing: 40 to 80% after 2 years
5. Conditions for viability test
   (1) Microorganism recovery: 30°C
   (2) Inoculation, cultivation, and confirmation method: Under the same conditions as the culture conditions.

As described above, the specific yeast of the present invention is preferably one that has been cultured in YPD medium at a pH of 4 to 7, preferably at a pH of 5 to 6. The culturing may be performed under aerobic conditions, for example, under atmospheric conditions.

The yeast of the present invention may be in the form of yeast cell walls, yeast intracellular contents, or the whole yeast cell. Yeast cell walls are fractions that are insoluble in aqueous media such as water, and are obtained by removing, from yeast cells, intracellular contents that dissolve in aqueous media, such as proteins, carbohydrates, amino acids, nucleic acids, and organic acids. The method for providing the yeast cell walls is not particularly limited, and the yeast cell walls may be provided by disrupting or breaking the yeast cells to render the water-soluble intracellular contents releasable, then obtaining the water-insoluble fraction by solid-liquid separation, and subsequently drying this fraction. Examples of methods for disruption or breakage include: physical disruption methods such as ultrasonic treatment, grinding methods using a bead mill, and pressurized liquid shear methods such as a French press; and chemical disruption methods using surfactants or lytic enzymes such as cell wall-degrading enzymes. As for drying methods, any method may be employed, such as freeze-drying or spray-drying. As a measure to render the intracellular contents of the yeast cells releasable in an aqueous medium, physical disruption is preferred. Examples of the aqueous medium here include water, ethanol, and mixtures thereof, and water is preferable. When the aqueous medium includes water, the water may contain any components other than water, and the water content may exceed 50 mass%, preferably 60 mass% or more.

Yeast intracellular contents can be obtained by disrupting yeast cells to render the intracellular contents releasable in an aqueous medium, and then extracting the resulting disrupted material with an aqueous medium. Instead of disrupting the yeast cells, the yeast cell walls may be dissolved with a cell wall-degrading enzyme to render the intracellular contents releasable in an aqueous solvent. The intracellular contents of the yeast are also referred to as "yeast extract". The yeast extract may be a solid obtained by drying using any suitable method.

Among the yeast cell walls and the yeast intracellular contents, a yeast component that contains the yeast intracellular contents is preferably used in the present invention, because it has superior immunomodulatory effects, specifically immunoenhancing and immunosuppressive effects. Examples of the yeast component that contains the yeast intracellular contents include the yeast intracellular contents themselves and also mixtures obtained by disrupting or breaking yeast cells and thus containing yeast cell walls and intracellular contents of the yeast in a mixed state. Such a mixture can be brought into contact with an aqueous liquid and subjected to solid-liquid separation to separate the yeast extract and the cell walls from each other. The proportion of the yeast extract in the yeast component is preferably 10 mass% or more, more preferably 20 mass% or more, even more preferably 50 mass% or more, and particularly preferably 60 mass% or more.

There is no critical limitation on the dosage when the specific yeast, which is the active ingredient of the immunomodulatory agent of the present invention, is used. Since the effects to be obtained vary depending on various modes when use, such as the subject and the disease to be treated, it is desirable to appropriately set the dosage. A preferred dosage of the specific yeast is 10 mg to 100 g per day in terms of cell weight, and more preferably 160 mg to 4 g.

The agent of the present invention can be taken continuously and may be taken continuously for one week or more, two weeks or more, or four weeks or more.

Examples of routes of intake of the specific yeast of the present invention include oral, transdermal, transmucosal, and enteral intake, and oral intake is particularly preferred.

The immunomodulatory agent of the present invention can be used as a pharmaceutical, quasi-drug, or food or beverage for animals, including humans, or for producing any of these. The immunomodulatory agent of the present invention may be directly administered to or taken by animals, including humans, as a pharmaceutical, quasi-drug, or food or beverage, or may be added to or incorporated into food or beverage, or animal feed such as pet food, to use the resultant as an immunomodulatory food, beverage, or animal feed. In the latter case, the method of adding or incorporating the specific yeast into a food, beverage, or animal feed is not particularly limited. For example, the specific yeast may be directly blended with raw materials or ingredients before the production of the food, beverage, or animal feed; the specific yeast may be added during the production process of the food, beverage, or animal feed; or the specific yeast may be added to the produced food, beverage, or animal feed.

The term "food or beverage" above refers to substances that can be consumed as food by humans. Examples thereof include general food and beverages, including so-called health foods, as well as foods with health claims, such as foods for specified health uses and foods with nutrient function claims as defined under the Food with Health Claims system of the Ministry of Health, Labour and Welfare, Japan, and also supplements.

The term "animal feed" above refers to substances given as feed to non-human animals (animals raised by humans), such as livestock, poultry, and farmed fish, and examples thereof include livestock feed and pet food.

When the immunomodulatory agent of the present invention is used as a pharmaceutical or quasi-drug, it may contain the specific yeast, which is the active ingredient, alone; it may further contain a pharmaceutically acceptable excipient; or it may further contain other active ingredients or pharmacologically active components, as long as the immunomodulatory effect of the specific yeast is not impaired. Examples of the excipient include carriers, coating agents, binders, extenders, disintegrants, surfactants, lubricants, diluents, dispersants, buffers, osmotic pressure regulators, pH regulators, emulsifiers, preservatives, stabilizers, antioxidants, colorants, UV absorbers, humectants, thickeners, activity enhancers, antimicrobial agents, flavoring agents, and deodorizing agents.

When the agent of the present invention is used as a pharmaceutical or quasi-drug, it may be administered in any administration form. The administration form may be oral or parenteral. Examples of oral administration forms include solid dosage forms such as tablets, coated tablets, granules, powders, and capsules, and liquid dosage forms such as elixirs, syrups, and suspensions. Examples of parenteral administration forms include injection, infusion, transdermal, transmucosal, intranasal, enteral, inhalation, suppositories, boluses, and patches. Among these, oral administration forms are preferred.

In the agent of the present invention, the amount of the specific yeast may be arbitrary, as long as the amount allows the specific yeast to serve as an active ingredient. The specific yeast may account for 5 mass% or more, or 10 mass% or more, or 20 mass% or more, or 30 mass% or more, or 40 mass% or more, or 50 mass% or more, or 70 mass% or more of the solid content in the agent. Note that the term "solid content" here refers to the total amount excluding the solvent, and examples of the solvent include water, ethanol, and organic solvents commonly used as solvents.

When the agent of the present invention is used as a food or beverage, it may contain the specific yeast, which is the active ingredient, alone; or it may further contain various additives used in the production of foods and beverages, provided that the immunomodulatory effect of the specific yeast is not impaired. Examples of such additives include various oils and fats, herbal medicines, amino acids, polyhydric alcohols, natural polymers, vitamins, dietary fibers, surfactants, purified water, carriers, stabilizers, pH regulators, antioxidants, sweeteners, flavoring components, acidulants such as organic acids, stabilizers, flavors, colorants, and fragrances.

Examples of the foods and beverages include oral preparations (such as gums and candies); processed fish paste products such as *kamaboko* and *chikuwa*; livestock products such as sausages and hams; bread; Western-style and Japanese-style confectioneries; noodles such as fresh noodles, Chinese noodles, boiled noodles, and buckwheat noodles; seasonings such as sauces, soy sauce, seasoning liquids, sugar, honey, powdered syrup, and starch syrup; spices such as curry powder, mustard powder, and pepper powder; jams, marmalades, and chocolate spreads; processed vegetables and fruits such as pickles, ready-prepared foods, *furikake* (flaked seasonings for sprinkling over rice, etc.), and canned or jarred vegetables and fruits; dairy products such as cheese, butter, and yogurt; miso soup and other soups; beverages such as fruit juices, vegetable juices, whey beverages, soft drinks, and alcoholic beverages; and other general foods and beverages including health foods.

The present invention also encompasses a package including a packaging material and the above-described immunomodulatory agent of the present invention or a food, beverage, or animal feed containing the immunomodulatory agent, wherein the agent, or the food, beverage, or animal feed is enclosed in the packaging material.

The packaging material may be any type of packaging material, as long as the agent of the present invention, or the food, beverage, or animal feed, can be enclosed therein and a list of ingredients and other information of the agent, food, beverage, or animal feed can be printed thereon, and the form and material of the packaging material are not particularly limited. Examples of the form of the packaging material include boxes and bags. Examples of the material of the packaging material include paper, plastic, woven fabric, and metal.

Various information, such as the content of the specific yeast in the agent of the present invention, food, beverage, or animal feed enclosed in the packaging material, is displayed on the packaging material. The method of displaying the information on the packaging material is not particularly limited. For example, 1) the information may be printed on the outer or inner surface of the packaging material; 2) the indication for immunomodulatory use may be printed on a printable medium, such as paper for printing, enclosed in the packaging material along with the food, beverage, or animal feed; or 3) a QR code (registered trademark) containing an Internet URL or information that allows access to a website introducing the agent of the present invention, food, beverage, or animal feed may be printed on the packaging material or on a printable medium enclosed therein, such that the information is displayed when the website is accessed via the URL.

As demonstrated in the Examples described later, the specific yeast exhibits an excellent effect in regulating cytokine production from cells such as macrophage cells or dendritic cells. The macrophage cells and dendritic cells are preferably those derived from mammals. Examples of mammals include humans, rats, mice, rabbits, cattle, pigs, dogs, cats, sheep, monkeys, and other non-human mammals. The macrophage cells and dendritic cells are mature cells capable of performing their inherent functions, such as phagocytic function and antigen-presenting function.

For example, as demonstrated in Examples 1 to 5 described later, the specific yeast of the present invention promotes cytokine production when administered to macrophage cells or dendritic cells, for example, macrophage cells or dendritic cells in a state where they have hardly been stimulated by antigens after differentiation. For example, the specific yeast of the present invention effectively enhances gene expression and promotes production of at least one cytokine selected from interleukin-1β (IL-1β), interleukin-6 (IL-6), interleukin-8 (IL-8), interleukin-10 (IL-10), interleukin-12 (IL-12), and TNF-α.

IL-1β, IL-6, IL-8, and TNF-α are proinflammatory cytokines. IL-1β is a potent proinflammatory cytokine. IL-1β stimulates CD4⁺ cells and induces their differentiation into Th17 cells. TNF-α plays an important role including inflammatory responses both locally and in the bloodstream. TNF-α not only induces the expression of vascular endothelial cells, but also enhances leukocyte adhesion molecules that stimulate immune cell infiltration, thereby promoting lymphocyte infiltration into the site of infection. IL-6 is important for inducing the differentiation of B cells into antibody-producing cells (plasma cells). IL-8 induces the chemotaxis of neutrophils and other granulocytes to the site of infection, and upon their arrival, IL-8 further induces phagocytosis.

IL-10 suppresses immune responses and also exhibits immunostimulatory properties, including stimulating the proliferation of thymocytes treated with IL-2 and IL-4, increasing the survival rate of B cells, and stimulating MHC class II expression.

IL-12 induces the production of interferon-gamma by natural killer cells (NK cells) and T cells, and enhances the activity of NK cells and cytotoxic T cells. The promotion of IL-12 production is evident from the promotion of production of its subunit, IL-12A.

In view of the foregoing, the specific yeast of the present invention can activate T cells, B cells, NK cells, and the like by exerting an effect of promoting the production of the aforementioned various cytokines, and can protect the living body against pathogens, viruses, and the like as a result of immune responses due to the effects of these various cytokines.

On the other hand, the specific yeast of the present invention can exhibit immunosuppressive effect on macrophage cells and dendritic cells that have already been stimulated and activated by antigens (such as lipopolysaccharide (LPS)). For example, the specific yeast of the present invention can suppress the production of proinflammatory cytokines, such as IL-1β, IL-6, and TNF-α, in macrophage cells.

LPS is an antigen that can strongly induce the expression of proinflammatory cytokines, such as IL-1β, IL-6, and TNF-α; however, excessive production of these proinflammatory cytokines may cause tissue damage and has been associated with various inflammatory diseases, such as rheumatoid arthritis and osteoarthritis, Alzheimer's disease, adult respiratory distress syndrome, allergies, asthma, eating disorders, and atherosclerosis, as well as muscle contusion, cancer, and others. Accordingly, the use of the specific yeast of the present invention can prevent these diseases.

It is also known that LPS exhibits endotoxin activity, and that TNF-α and IL-1 produced by macrophage cells in response to large amounts of LPS increase blood coagulation in blood vessels and vascular permeability, leading to decreased blood pressure to result in peripheral circulatory failure and shock (endotoxin shock). It is also known that when IL-1, IL-6, TNF-α, and other cytokines produced from various cells by the action of LPS are transported via the bloodstream to the brain, prostaglandin E2 is produced from brain cells and acts on the hypothalamic-pituitary axis, which regulates body temperature, leading to the onset of fever. Accordingly, the use of the specific yeast of the present invention may prevent or ameliorate such shock effects in blood vessels, fever, and the like.

It is known that when adipocytes become excessively large due to obesity, the adipocytes die, and immune cells such as macrophages that phagocytose the dead adipocytes accumulate in visceral adipose tissue, causing inflammation. This inflammation is considered to affect the entire body and lead to lifestyle-related diseases such as diabetes. Accordingly, the use of the specific yeast of the present invention can prevent diseases such as inflammation caused by obesity.

The specific yeast of the present invention can exhibit the effect of increasing IL-12 in macrophage cells and dendritic cells that have already been stimulated and activated by antigens (such as LPS). Increasing IL-12 production in macrophage cells and dendritic cells leads to enhanced expression of IL-10 in T cells, which in turn is thought to lead to anti-inflammation.

In addition, IL-12 is known to have a suppressive effect on IgE production, thereby suppressing Th2-type immune responses, which is thought to lead to the suppression of Th2-type immune responses.

Th2-type immune abnormalities are considered to be associated with allergic diseases (atopic dermatitis), myasthenia gravis, chronic active hepatitis (HBsAg(-)), membranous glomerulonephritis, pemphigus vulgaris, hyperthyroidism or hypothyroidism, and sarcoidosis, for example. They are also considered to lead to chronic rheumatoid arthritis, SLE, urticaria, and bronchial asthma, for example. Accordingly, the use of the specific yeast of the present invention can prevent these diseases.

As shown in Examples 10 and 11 described later, the specific yeast of the present invention selectively enhances the expression of TLR2 on the surface of cells, regardless of whether it is a mixture of cell walls and intracellular contents or the intracellular contents alone. TLR2 is reported to be expressed and function not only in cells such as dendritic cells and macrophages, which govern innate immunity, but also in T cells, which play a central role in adaptive immunity, and to play an important role in infection immunity, antitumor immunity, and the induction of autoimmune diseases. Accordingly, the cytokine production regulatory function of the specific yeast of the present invention is considered to be mediated by TLR2.

It is expected that the immunomodulatory agent of the present invention described above can bring about an immunomodulatory effect suitable for the physical condition at the time of intake. For example, it is considered that the immunoenhancing effect of suppressing infection by viruses or bacteria when immunity is reduced can be obtained by intake during fatigue or after exercise, for example. Accordingly, the immunomodulatory agent of the present invention can also be used as an immunoenhancer.

It can also be expected, for example, that intake of the immunomodulatory agent of the present invention during a cold, or intake by individuals who are obese or elderly, can bring about immunosuppressive effects such as an anti-inflammatory effect, thereby alleviating inflammation associated with colds and similar conditions, chronic inflammation, or autoimmune diseases. Accordingly, the immunomodulatory agent of the present invention can also be used as an anti-inflammatory agent or an immunosuppressant.

Whether an immunoenhancing effect or an immunosuppressive effect is exerted on the same individual may be dependent on the degree of inflammation in the individual, for example, the degree and condition of inflammation at the site of administration.

Examples of methods for evaluating the degree and condition of inflammation in mammals such as humans include inflammation markers in blood.

Conventional microbial strains that have been reported to affect immunity were generally known to exhibit only one of the effects, either immunosuppressive or immunoenhancing, whereas the specific yeast of the present invention has been found to exhibit both immunoenhancing and immunosuppressive effects. As such, the specific yeast of the present invention may exert an effect suitable for the physical conditions of the consumer. It is considered that the immunomodulatory agent of the present invention has the effect of enhancing immunity when immune function is reduced, and the effect of reducing immunity when immune function is increased. As such, the immunomodulatory agent of the present invention is considered to be capable of exerting immunosuppression without reducing immune strength. Furthermore, it is expected to control abnormalities in both decreases and increases in immune status occurring in an individual, thereby preventing the onset of pathological conditions or acting to return an existing pathological condition to normal. Accordingly, even when the immune status of the body changes during continuous intake, it may be capable of exerting an effect suitable for such change, and is excellent as an immunomodulatory agent.

For example, the present invention provides the following.
[1] An immunomodulatory agent containing yeast deposited with the National Institute of Technology and Evaluation under International Deposit No. NITE BP-04012.
[2] The immunomodulatory agent as set forth in clause [1], for regulating cytokine production.
[3] The immunomodulatory agent as set forth in clause [2], which regulates cytokine production in macrophage cells or dendritic cells.
[4] The immunomodulatory agent as set forth in any one of clauses [1] to [3], which modulates immunity via TLR2.
[5] The immunomodulatory agent as set forth in any one of clauses [2] to [3], wherein the cytokine is at least one selected from IL-1β, IL-6, IL-8, IL-10, IL-12, and TNF-α.
[6] The immunomodulatory agent as set forth in any one of clauses [1] to [5], for immunoenhancement and immunosuppression.
[7] The immunomodulatory agent as set forth in clause [6], which is used to exert an immunoenhancing effect or an immunosuppressive effect depending on a condition or degree of inflammation in a body.
[8] The immunomodulatory agent as set forth in any one of clauses [1] to [7], which is used to enhance production of at least one selected from IL-1β, IL-6, IL-8, IL-10, IL-12, and TNF-α.
[9] The immunomodulatory agent as set forth in any one of clauses [1] to [7], which is used to suppress production of at least one selected from IL-1β, IL-6, and TNF-α under an inflammatory condition or to enhance production of IL-12 under an inflammatory condition.
[10] The immunomodulatory agent as set forth in any one of clauses [1] to [7], which is used to regulate production of at least one selected from IL-1β, IL-6, IL-8, IL-10, IL-12, and TNF-α in macrophages or used to regulate production of IL-6 in dendritic cells.
[11] The immunomodulatory agent as set forth in any one of clauses [1] to [10], containing a cell wall component of the yeast.
[12] The immunomodulatory agent as set forth in any one of clauses [1] to [10], containing an intracellular content of the yeast.
[13] An immunomodulatory food or beverage containing the immunomodulatory agent as set forth in any one of clauses [1] to [12].
[14] Use of yeast deposited with the National Institute of Technology and Evaluation under International Deposit No. NITE BP-04012 for production of an immunomodulatory agent.

### Examples

The present invention will be described in further detail below by way of examples; however, the present invention is not limited to the examples given below. In the following tests, macrophage-like cells were used as a model for macrophage cells, and dendritic cell-like cells were used as a model for dendritic cells. The macrophage cells and dendritic cells in the present invention include the macrophage-like cells and dendritic cell-like cells described below, respectively.

### <Culture and Drying of Yeast >

The yeast strain (International Deposit No. NITE BP-04012) was cultured using the following method. First, a seed culture stored under the above-described conditions was collected, and the seed culture was streaked onto YPD agar medium. After culturing on the agar medium under atmospheric conditions at 30°C for 3 days, multiple colonies that appeared on the agar medium were cultured under the above-described conditions. After the culturing, the yeast was dried by spray drying.

### <Preparation of Yeast Powder>

First, 150 mg of the dried powder obtained in <Culture and Drying of Yeast> described above was suspended in 1.5 mL of sterile water, and the resulting suspension was placed in a tube. The cell walls were disrupted by grinding using a homogenizing system for cells with beads (Tomy Medico, Micro Smash (trademark) MS-100R), and the resultant was subsequently freeze-dried, thereby obtaining a disrupted product with the intracellular contents released. Then, 10 mg of the resulting solid was suspended in 10 mL of each test medium to prepare a yeast powder with the yeast intracellular contents released (concentration in the medium: 1 w/v%; hereinafter also referred to simply as "yeast powder"). The resulting yeast powder contains both yeast cell walls and yeast extract, and the proportion of the yeast extract was approximately 60 mass%.

### <Preparation of Yeast Extract>

10 mL of each test medium was added to 10 mg of the yeast powder (disrupted product) obtained in <Preparation of Yeast Powder> described above. The mixture was thoroughly stirred, and then precipitation was allowed to form. The resulting supernatant was collected and used as the extract (concentration in the medium: 1 w/v%; hereinafter also referred to simply as "yeast extract").

### <Evaluation 1: Evaluation of Cytokine Production Level (Gene Expression, Macrophage-like Cells)>

THP-1 cells (human monocyte-derived cells) were cultured in a growth medium (RPMI 1640 medium supplemented with 10% FBS (fetal bovine serum)), and after 4 days, the cells were seeded in a differentiation induction medium (RPMI 1640 medium supplemented with 10% FBS and 0.5 µg/mL PMA (Phorbol 12-myristate 13-acetate)) at 1×10⁴ cells/0.1 mL/well, in a 96-well plate. After confirming differentiation into macrophage-like cells after 4 days of culturing, the medium was replaced with a test medium (RPMI 1640 medium supplemented with 10% FBS, 100 Units/mL penicillin, and 100 Units/mL streptomycin) containing the test substance at each concentration or containing LPS (Sigma-Aldrich) and the test substance, and the cells were cultured under conditions of 37°C and 5% CO₂ for 1 hour. The medium was then replaced again with a test medium containing the test substance or containing LPS and the test substance, followed by culturing for 5 hours.

The cells were washed twice with PBS, and cDNA was collected using a FastLane cDNA kit (QIAGEN). Using the collected cDNA, real-time PCR reactions were performed with TB Green (registered trademark) Premix Ex Taq (trademark) II (Takara Bio Inc.). A relative gene expression level was calculated by comparative quantification using the ΔΔCt method relative to the Ct value, which is the expression level of an internal reference gene. The primer sequences for each gene are shown in Table 1 below.

**[Table 1]**

| Gene | | Sequence (5'→3') |
|---|---|---|
| IL-1β | Forward | ATGATGGCTTATTACAGTGGCAA |
| | Reverse | GTCGGAGATTCGTAGCTGGA |
| IL-6 | Forward | ACTCACCTCTTCAGAACGAATTG |
| | Reverse | CCATCTTTGGAAGGTTCAGGTTG |
| IL-8 | Forward | ATGACTTCCAAGCTGGCCGTGGC |
| | Reverse | TCTCAGCCCTCTTCAAAAACTTC |
| IL-10 | Forward | GACTTTAAGGGTTACCTGGGTTG |
| | Reverse | TCACATGCGCCTTGATGTCTG |
| IL-12A | Forward | GAGACTGTGCCACAAAAATCCTCCC |
| | Reverse | TCACTCTATCAATAGTCACTGCCCG |
| TNF-α | Forward | GAGGCCAAGCCCTGGTATG |
| | Reverse | CGGGCCGATTGATCTCAGC |
| GAPDH (Internal reference) | Forward | CATCCCTGCCTCTACTGGCGCTGCC |
| | Reverse | CCAGGATGCCCTTGAGGGGGCCCTC |

### <Evaluation 2: Evaluation of Cytokine Production Level (Gene Expression, Dendritic Cell-like Cells)>

THP-1 cells (human monocyte-derived cells) were cultured in a growth medium (DMEM medium supplemented with 10% FBS (fetal bovine serum)), and after 2 to 3 days, the cells were seeded in a differentiation induction medium (DMEM medium supplemented with 200 ng/mL ionomycin, 100 ng/mL GM-SCF, 200 ng/mL IL-4, and 20 ng/mL TNF-α) at 5×10⁵ cells/0.5 mL/well, in a 24-well plate. After culturing for 48 hours to induce differentiation into dendritic cell-like cells, a diluent was added to the plate in an amount of 3.3 µL/well so that the test substance reached the specified concentration, followed by culturing under conditions of 37°C and 5% CO₂ for 24 hours. Sterile water was used as the diluent here.

The cells were washed with PBS, and RNA was extracted using a commercially available kit (Takara Bio Inc.). cDNA was collected using a PrimeScript (trademark) RT reagent Kit with gDNA Eraser (Takara Bio Inc.), and real-time PCR reactions were performed with TB Green (registered trademark) Premix Ex Taq (trademark) II. A relative gene expression level was calculated by comparative quantification using the ΔΔCt method relative to the Ct value, which is the expression level of an internal reference gene. The primer sequences for each gene are shown in Table 2 below.

**[Table 2]**

| Gene | | Sequence (5'→3') |
|---|---|---|
| IL-6 | Forward | CCAGAACAGATTTGAGAG |
| | Reverse | TCGTCAGCAGGCTGGCAT |
| 18S rRNA (Internal reference) | Forward | CGGCGACGACCCATTCGAAC |
| | Reverse | GAATCGAACCCTGATTCCCCGT |

### <Evaluation 3: Evaluation of Cytokine Production Level (ELISA Analysis, Macrophage-like Cells)>

After culturing THP-1 in a growth medium, the cells were seeded in a macrophage-like differentiation induction medium at 6.3×10⁴ cells/0.5 mL/well, in a 24-well plate. The cells were cultured under conditions of 37°C and 5% CO₂ for 4 days. After confirming differentiation into macrophage-like cells, the medium was replaced with a test medium (RPMI 1640 medium supplemented with 10% FBS, 100 Units/mL penicillin, and 100 Units/mL streptomycin) containing the test substance at each concentration or containing LPS (Nacalai Tesque, Inc.) and the test substance, and the cells were cultured under conditions of 37°C and 5% CO₂ for 1 hour. The medium was then replaced again with a test medium containing the test substance or containing LPS and the test substance, followed by culturing for 24 hours. The culture supernatant from each well was transferred to a 1.5-mL tube and centrifuged at 3,000 ×g for 5 minutes, and the resulting supernatant was used for ELISA analysis. In the ELISA analysis, IL-1β was measured using an IL-1β ELISA Kit, Human (ProteinTech Group, Inc.), IL-6 was measured using a Rebis (registered trademark) Human IL-6 ELISA Kit (FUJIFILM Wako Shibayagi Corporation), IL-8 was measured using a Rebis (registered trademark) Human IL-8 ELISA Kit (FUJIFILM Wako Shibayagi Corporation), IL-10 was measured using an AuthentiKine (trademark) IL-10 ELISA Kit, Human (ProteinTech Group, Inc.), IL-12A was measured using a Human IL-12 ELISA Kit (R&D Systems, Inc.), and TNF-α was measured using an AuthentiKine (trademark) TNF-alpha ELISA Kit, Human (ProteinTech Group, Inc.).

### <Evaluation 4: Evaluation of TLR Receptor Activation>

In this evaluation, HEK293 cells were used as cells capable of highly efficient gene transfection.

An NF-κB Reporter Luciferase HEK293 Cell Line was seeded in a growth medium (DMEM medium supplemented with 10% FBS (fetal bovine serum), 100 U/mL penicillin, 100 U/mL streptomycin, and 100 µg/mL hygromycin B) at 1×10⁴ cells/100 µL/well, in a 96-well plate, and after culturing for 24 hours, a plasmid vector having a TLR2 or TLR4 gene (TLR2: pRP[Exp]-Neo-CMV>hTLR2 (Vector Builder Inc.), TLR4: pRP(Exp)-Neo-CMV>hTLR4 (Vector Builder Inc.)) was introduced into the cells by lipofection. A transfection solution was prepared by mixing 100 ng of plasmid DNA per well, 0.1 µL of X-tremeGENE (trademark) HP DNA transfection reagent (MERCK), and 10 µL of DMEM medium, and the transfection solution was added in an amount of 10 µL/well, followed by mixing gently and culturing again. On the following day, as confirmation of vector introduction, the cells were further cultured for one day in a test medium containing 200 µg/mL G418 (RPMI 1640 medium supplemented with 10% FBS, 100 Units/mL penicillin, 100 Units/mL streptomycin, and 200 µg/mL G418).

The culture supernatant in each well was then replaced with a test medium containing 100 µL/well of the test substance or a positive control (10 µg/mL zymosan or 1 µg/mL LPS). The cells were then cultured for 6 hours, and after completion of the culture, luciferase activity in the cells was evaluated by measuring luminescence using a Bright-Glo (trademark) Luciferase Assay System (Promega Corporation). After completion of the culture, 30 µL of the culture supernatant was taken from each well, and 70 µL of Luciferase Assay Reagent was added thereto. After gentle mixing and standing for 2 minutes, the relative luminescence value of each well was measured using a microplate reader. The relative luminescence value was expressed as the ratio to a control (to which neither the test substance nor the positive control was added).

### (Example 1: Increase in Cytokine Gene Expression Levels (Macrophage-like Cells))

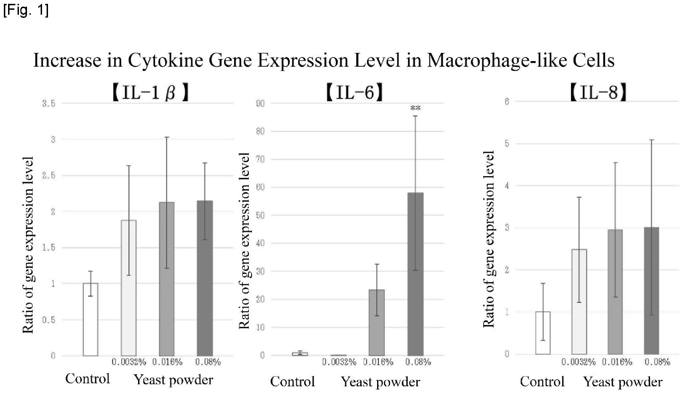

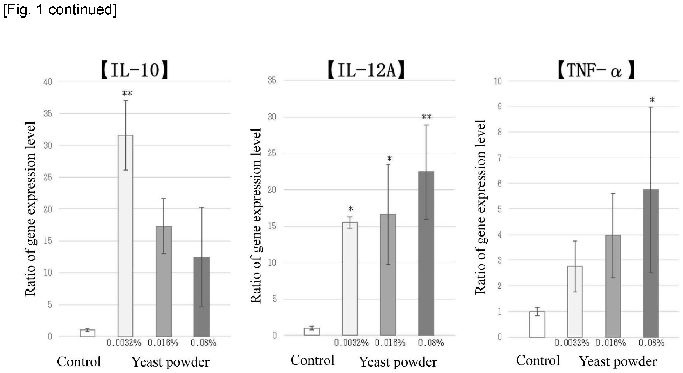

For macrophage-like cells, <Evaluation 1: Evaluation of Cytokine Production Level (Gene Expression, Macrophage-like Cells)> described above was performed by adding the above-described yeast powder as the test substance, and the cytokine gene expression level (relative values to an internal reference) was thus determined. The ratio of the found gene expression level to the cytokine gene expression level of a control, which was determined in the same manner except that no yeast powder was added, was calculated. Fig. 1 shows the results.

As shown in Fig. 1, it was confirmed that for various cytokine genes, the addition of the yeast powder increased the expression level of the cytokine gene compared to the case where no yeast powder was added.

### (Example 2: Increase in Cytokine Gene Expression Levels (Macrophage-like Cells))

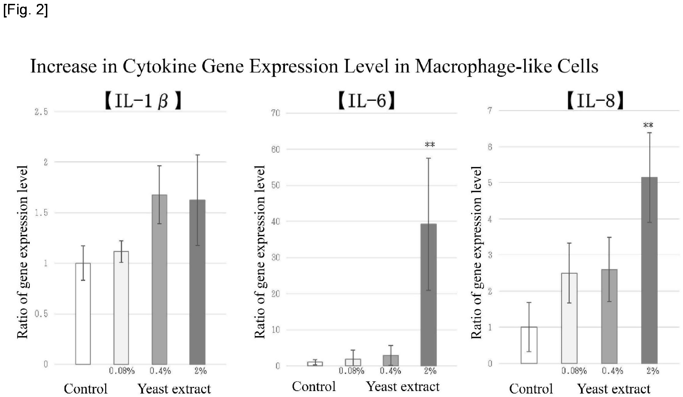

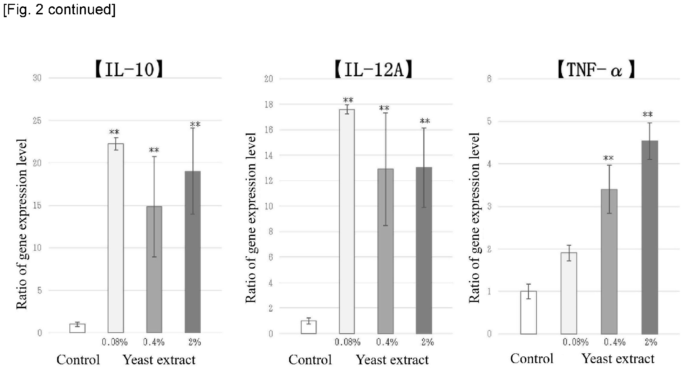

For macrophage-like cells, <Evaluation 1: Evaluation of Cytokine Production Level (Gene Expression, Macrophage-like Cells)> described above was performed by adding the above-described yeast extract as the test substance, and the cytokine gene expression level (relative values to an internal reference) was thus determined. The ratio of the found gene expression level to the cytokine gene expression level of a control, which was determined in the same manner except that no yeast extract was added, was calculated. Fig. 2 shows the results.

As shown in Fig. 2, it was confirmed that for various cytokine genes, the addition of the yeast extract increased the expression level of the cytokine gene compared to the case where no yeast extract was added.

### (Example 3: Increase in Cytokine Gene Expression Level (Dendritic Cell-like Cells))

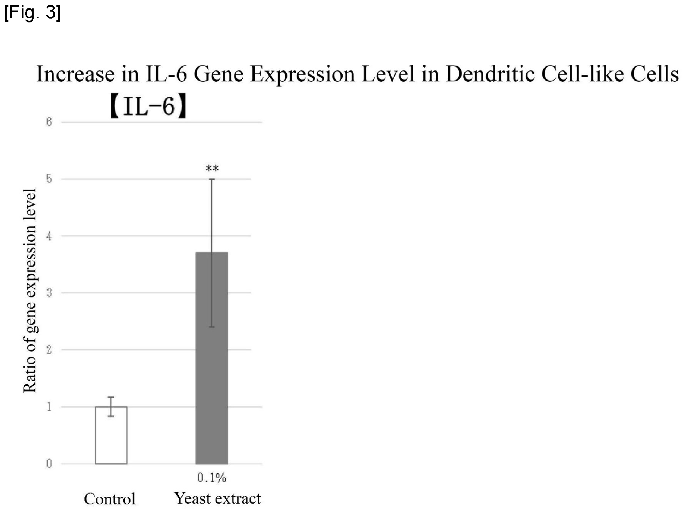

For dendritic cell-like cells, <Evaluation 2: Evaluation of Cytokine Production Level (Gene Expression, Dendritic Cell-like Cells)> described above was performed by adding the above-described yeast extract as the test substance, and the IL-6 cytokine gene expression level was thus determined. The ratio of the found gene expression level to the cytokine gene expression level of a control, which was determined in the same manner except that no yeast extract was added, was calculated. Fig. 3 shows the results.

As shown in Fig. 3, it was confirmed in dendritic cell-like cells as well that the cytokine gene expression level was increased compared to the case where no addition was made.

### (Example 4: Increase in Cytokine Production Levels)

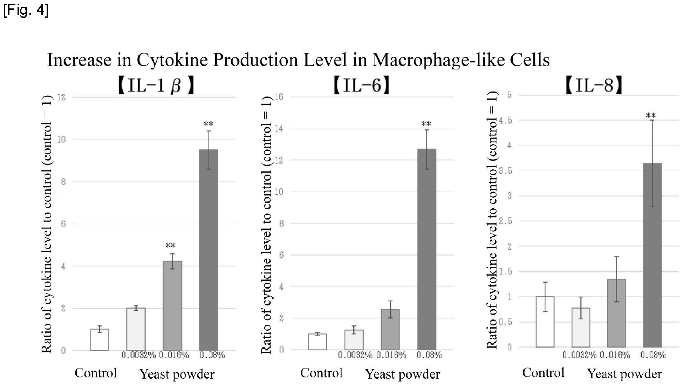

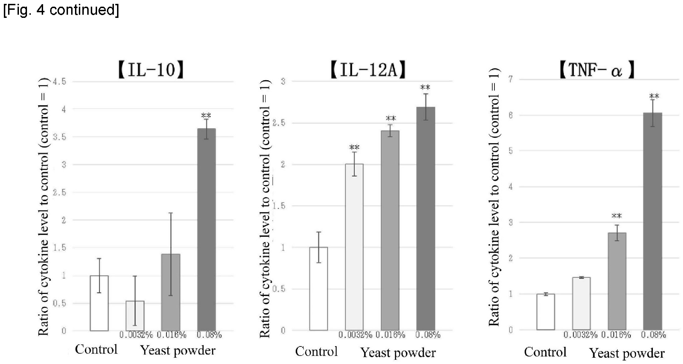

For macrophage-like cells, <Evaluation 3: Evaluation of Cytokine Production Level (ELISA Analysis, Macrophage-like Cells)> described above was performed by adding the above-described yeast powder as the test substance, and the cytokine production level was thus determined by ELISA analysis. The ratio thereof to the cytokine production level of a control, which was determined in the same manner except that no yeast powder was added, was calculated. Fig. 4 shows the results. As shown in Fig. 4, it was confirmed that for various cytokines, the addition of the yeast powder increased the cytokine level compared to the case where no yeast powder was added.

### (Example 5: Increase in Cytokine Production Levels)

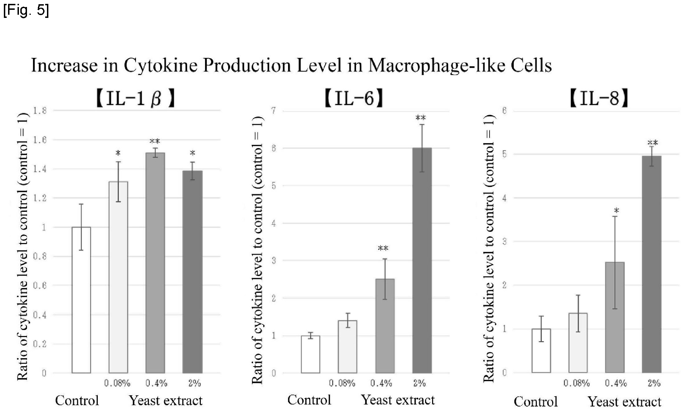

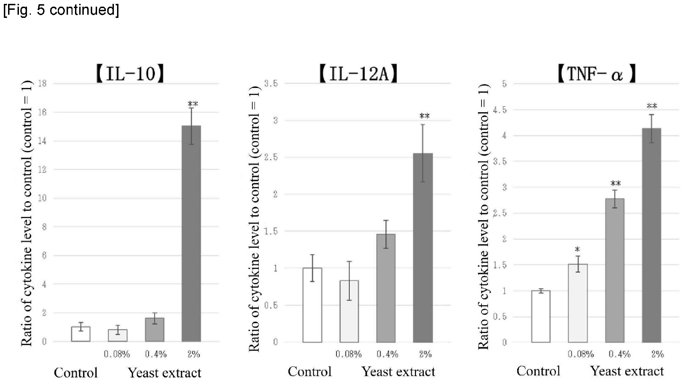

For macrophage-like cells, <Evaluation 3: Evaluation of Cytokine Production Level (ELISA Analysis, Macrophage-like Cells)> described above was performed by adding the above-described yeast extract as the test substance, and the cytokine production level was thus determined by ELISA analysis. Fig. 5 shows the results. As shown in Fig. 5, it was confirmed that for various cytokines, the addition of the yeast extract increased the cytokine level compared to the value of the control without addition.

### (Example 6: Regulation of Cytokine-Producing Gene Expression Levels)

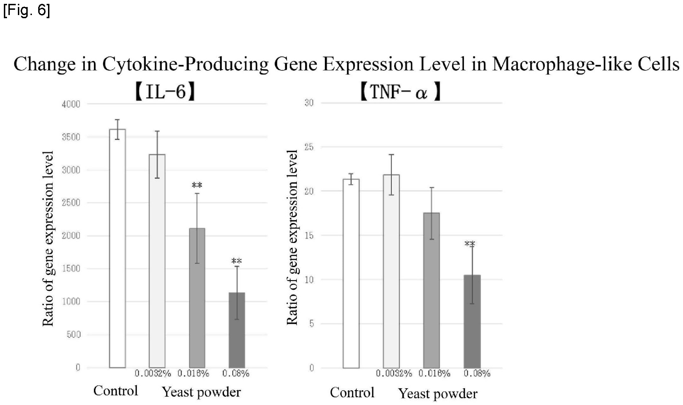

For macrophage-like cells, <Evaluation 1: Evaluation of Cytokine Production Level (Gene Expression, Macrophage-like Cells)> described above was performed by adding the above-described yeast powder as the test substance in addition to LPS 1 µg/mL, and the cytokine gene expression level (relative value to an internal reference) was thus determined. An inflammatory condition in the presence of LPS 1 µg/mL was used as a control. Fig. 6 shows the ratios of gene expression levels when the values obtained in the absence of both LPS and the test substance were defined as 1. It was confirmed that the addition of the yeast powder reduced the gene expression levels of proinflammatory cytokines (IL-6 and TNF-α) that increased under the inflammatory condition.

### (Example 7: Regulation of Cytokine-Producing Gene Expression Levels)

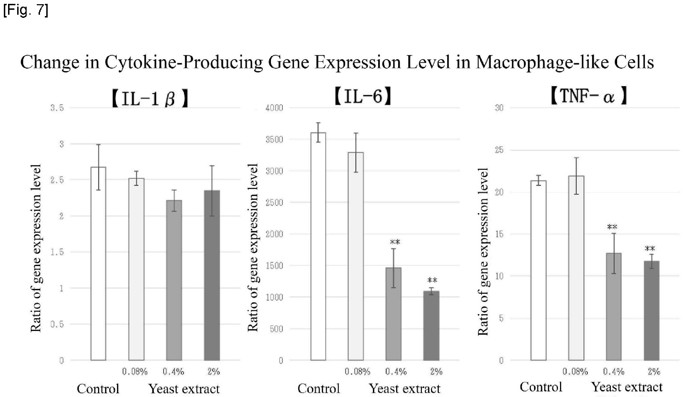

For macrophage-like cells, <Evaluation 1: Evaluation of Cytokine Production Level (Gene Expression, Macrophage-like Cells)> described above was performed by adding the yeast extract in addition to the LPS 1 µg/mL, and the cytokine gene expression level was thus determined. An inflammatory condition in the presence of LPS 1 µg/mL was used as a control. Fig. 7 shows the ratios of gene expression levels when the values obtained in the absence of both LPS and the test substance were defined as 1. It was confirmed that the addition of the yeast extract reduced the gene expression levels of proinflammatory cytokines that increased under the inflammatory condition.

### (Example 8: Regulation of Cytokine Production Levels)

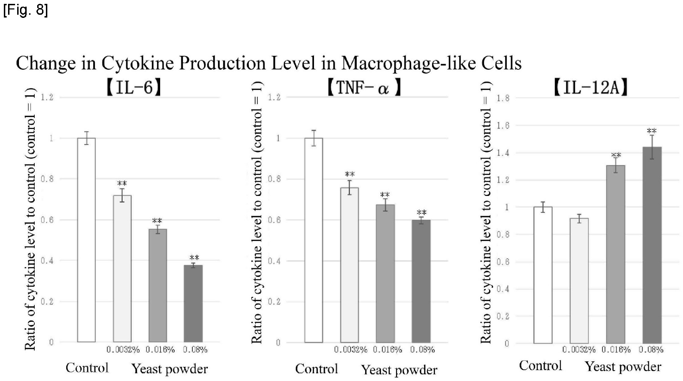

For macrophage-like cells, <Evaluation 3: Evaluation of Cytokine Production Level (ELISA analysis, Macrophage-like Cells)> described above was performed by adding the above-described yeast powder as the test substance in addition to the LPS 1 µg/mL, and the cytokine production level was thus determined by ELISA analysis. An inflammatory condition in the presence of LPS 1 µg/mL was used as a control. Fig. 8 shows the results. As shown in Fig. 8, it was confirmed that the addition of the yeast powder reduced the production levels of proinflammatory cytokines (IL-6 and TNF-α) under the inflammatory condition.

### (Example 9: Regulation of Cytokine Production Levels)

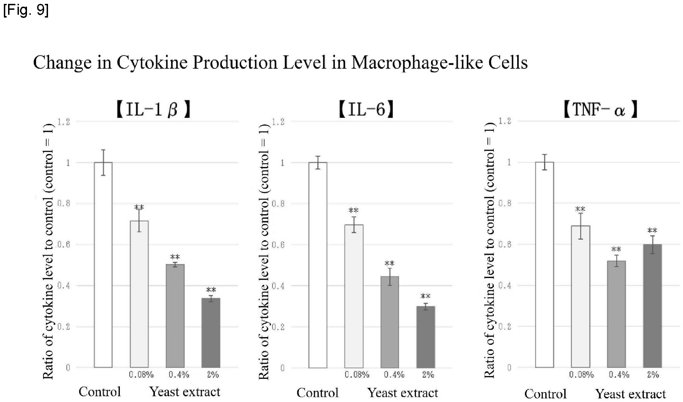

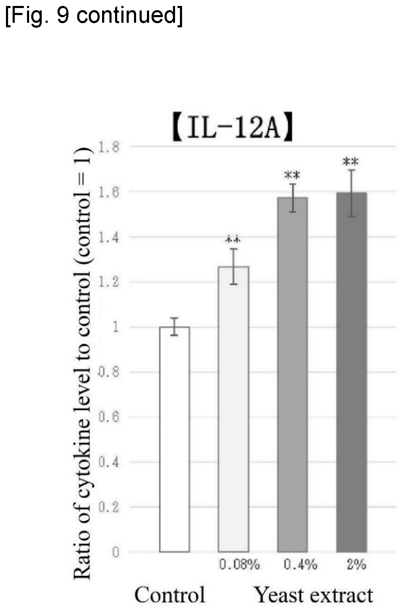

For macrophage-like cells, <Evaluation 3: Evaluation of Cytokine Production Level (ELISA Analysis, Macrophage-like Cells)> described above was performed by adding the above-described yeast extract as the test substance in addition to the LPS 1 µg/mL, and the cytokine production level was thus determined by ELISA analysis. An inflammatory condition in the presence of LPS 1 µg/mL was used as a control. Fig. 9 shows the results. As shown in Fig. 9, it was confirmed that the addition of the yeast extract reduced the production levels of proinflammatory cytokines (IL-6, TNF-α, and IL-1β) under the inflammatory condition.

### (Example 10: Activation of TLR Receptors)

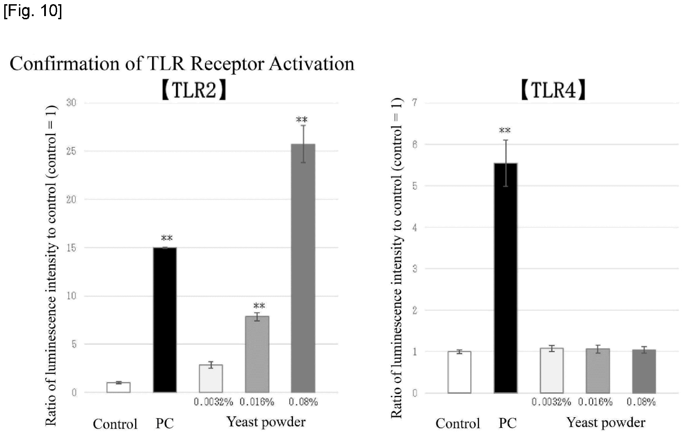

The activities of TLR2 and TLR4 receptors when the above-described yeast powder was added as the test substance were evaluated according to <Evaluation 4: Evaluation of TLR Receptor Activation>. As positive controls (PCs), zymosan (Nova Biologicals Inc.) was used for TLR2, and LPS-EB (Nacalai Tesque, Inc.) was used for TLR4. Fig. 10 shows the results. As shown in Fig. 10, it was confirmed that the addition of the yeast powder increased luciferase activity mediated by TLR2, indicating that the yeast powder increases the cytokine production level via TLR2.

### (Example 11: Activation of TLR Receptors)

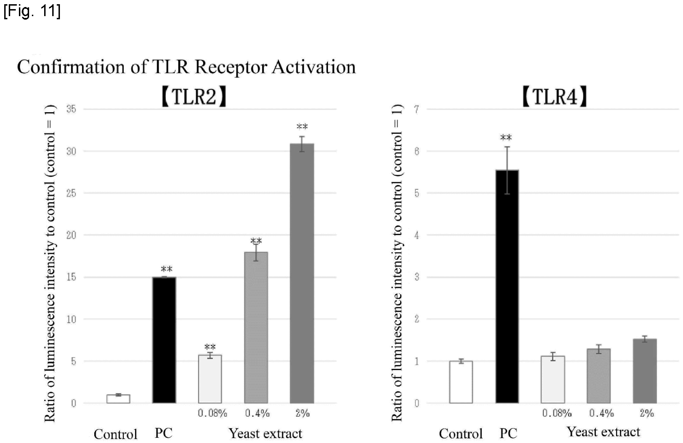

The activities of TLR2 and TLR4 receptors when the yeast extract alone was added were evaluated according to <Evaluation 4: Evaluation of TLR Receptor Activation>. As PCs (positive controls), zymosan (Nova Biologicals Inc.) was used for TLR2, and LPS-EB (Nacalai Tesque, Inc.) was used for TLR4. Fig. 11 shows the results. In Fig. 11, it was confirmed that the addition of the yeast extract increased luciferase activity mediated by TLR2, indicating that the yeast extract increases the cytokine production level via TLR2.

## Claims

1. An immunomodulatory agent comprising yeast deposited with the National Institute of Technology and Evaluation under International Deposit No. NITE BP-04012.

2. The immunomodulatory agent according to claim 1, for regulating cytokine production.

3. The immunomodulatory agent according to claim 2, which regulates cytokine production in macrophage cells or dendritic cells.

4. The immunomodulatory agent according to claim 1 or 2, which modulates immunity via TLR2.

5. The immunomodulatory agent according to claim 2, wherein the cytokine is at least one selected from IL-1β, IL-6, IL-8, IL-10, IL-12, and TNF-α.

6. The immunomodulatory agent according to claim 1 or 2, for immunoenhancement and immunosuppression.

7. The immunomodulatory agent according to claim 6, which is used to exert an immunoenhancing effect or an immunosuppressive effect depending on a condition or degree of inflammation in a body.

8. The immunomodulatory agent according to claim 1 or 2, which is used to enhance production of at least one selected from IL-1β, IL-6, IL-8, IL-10, IL-12, and TNF-α.

9. The immunomodulatory agent according to claim 1 or 2, which is used to suppress production of at least one selected from IL-1β, IL-6, and TNF-α under an inflammatory condition or to enhance production of IL-12 under an inflammatory condition.

10. The immunomodulatory agent according to claim 1 or 2, comprising a cell wall component of the yeast.

11. The immunomodulatory agent according to claim 1 or 2, comprising an intracellular content of the yeast.

12. An immunomodulatory food or beverage comprising the immunomodulatory agent according to claim 1 or 2.

13. Use of yeast deposited with the National Institute of Technology and Evaluation under International Deposit No. NITE BP-04012 for production of an immunomodulatory agent.
